# EUROPÄISCHE PATENTSCHRIFT

(11) **EP 1 881 058 B1**
(45) Veröffentlichungstag und Bekanntmachung des Hinweises auf die Patenterteilung: **05.01.2011**
(21) Anmeldenummer: 07014212.0
(22) Anmeldetag: 19.07.2007
(51) Int. Cl.: C11D 3/00, C11D 17/04, A61K 8/02, D06M 15/263, D06M 16/00, A01N 25/34

(54) **Reinigungstuch**
Cleaning cloth
Gant de nettoyage

(30) Priorität: 21.07.2006 DE 102006339347
(43) Veröffentlichungstag der Anmeldung: 23.01.2008
(73) Patentinhaber: Smart Fiber AG, 07407 Rudolstadt (DE)
(72) Erfinder: Kolbe, Axel, 07924 Neundorf (DE); Markwitz, Hardy, Dr.,, 07407 Rudolstadt (DE)
(74) Vertreter: Prietsch, Reiner

(56) Entgegenhaltungen:
- EP-A- 0 905 289
- EP-A- 1 614 790
- EP-A- 1 927 286
- WO-A-2005/026424
- WO-A-2006/013378
- DE-A1- 10 140 772
- GB-A- 2 381 270

## Beschreibung

### Gebiet der Erfindung

Die Erfindung betrifft ein Reinigungstuch mit microbiocider, insbesondere antibakterieller und fungizider Wirkung.

### Stand der Technik

Es ist bekannt, dass ein Wischtuch mit Wasserstoffperoxid (W02005040328), Aminoxidtensid, N-Alkylpyrrolidone-Derivat (WO2004074417) oder Alkali- und Ammoniumsulfonaten (W003102122) getränkt und dadurch antibakteriell ausgerüstet werden kann. Diese Produkte sind für Einmalanwendungen bestimmt. In eine Sandwichstruktur eingelagertes Hypochlorit wirkt ebenfalls desinfizierend (WO0147705). Das freiwerdende Chlordioxid ist ein sehr starkes und für den häuslichen Gebrauch nur bedingt einsetzbares Desinfektionsmittel. Polyurethanschwämme können mit quartären Ammoniumverbindungen bakterizid ausgestattet werden (EP-A-1599568). Schäume sind nicht universell einsetzbar und nicht wärmeresistent.

Schwermetallionen wie z. B. Silber-, Quecksilber-, Kupfer-, Zink- und Zirkoniumionen wirken auf Mikroorganismen wie Bakterien, Viren, Pilze und Sporen abtötend oder wachstumshemmend (Thurman et. al., CRC Crit. Rev. in Environ. Contr. 18 (4), S. 295-315 (1989)). Für eine microbiocide Wirkung sind Silberionen von besonderem Interesse. Der entscheidende Vorteil von Silberionen gegenüber anderen bakterizid wirkenden Metallionen, wie z. B. Hg²⁺, ist die weitestgehende Unempfindlichkeit des menschlichen Metabolismus gegenüber Silber. Die bakterizid wirkende Konzentration wird bei Silber mit 0,01 - 1 mg/l angegeben (Ullman's Encyclopedia of Industrial Chemistry (5. Aufl.), VCH 1993, Volume A 24, S. 160). Diese Wirkung der Silberionen wird seit langem in den unterschiedlichsten Anwendungen genutzt. Bei der Herstellung von textilen Fasern wird Silber z. B. galvanisch auf der Oberfläche von Polyamidseide abgelagert (Noble Fiber Technologies, http://www.x-static.com). Die Verarbeitung von galvanisch versilberter Polyamidseide auf Strick- und Wirkmaschinen ist problematisch, da sich an den Fadenleitorganen die Silberschicht der Polyamidseide teilweise ablagert und damit zu häufigen Maschinenstillständen führt. Eine weitere bekannte Möglichkeit ist das Einbringen von metallischem Silber, Silber-Zeolith oder Silberglaskeramik in die Fasermatrix von schmelzegesponnenen Fasern wie Polypropylenfasern, Polyesterfasern und Polyamidfasern (Taschenbuch für die Textil-Industrie 2003, Schiele & Schön Berlin, S. 124 ff). Silberionen, gebunden an Titandioxid, werden auch in Polyurethanschäumen in Haushaltsreinigungsmaterialien eingesetzt (EP-A-0990021).

Aus der GB-A-2 372 939 ist ein Haushaltstuch aus einem Trägergewebe mit einer Beimischung eines silberhaltigen Kunststoffgarns bekannt. Weiterhin ist bekannt, dass man Zeolithe als Trägermaterial für Silber, Kupfer und Zink einsetzen kann (EP-A-0116865, JP-A-4126820, W09859026).
Silberverbindungen können auch direkt in Cellulose eingearbeitet werden (EP-A-0905289), sind jedoch nicht waschfest gebunden.

Auch für Acrylnitrilfasern wurde der Einsatz von Silber-Zeolith und Silberglaskeramik vorgeschlagen. Als wärmeresistente Material mit einer bakteriziden Ausstattung ist bisher als Trägermaterial ausschließlich Polyacrylnitril bekannt (JP-A-11349423).

Auch cellulosische Fasern mit bakteriostatischen oder bakteriziden Eigenschaften sind bekannt. Durch Inkorporieren von Triclosan (2,4,4-Trichloro(II)-hydroxyphenyläther) in cellulosische Fasern erhält man eine permanent bakterio-statische Faser (ITB International Textile Bulletin 3/2002). Diese Substanz ist gegen Bakterien wirksam, die üblicherweise auf der Haut vorkommen, einschließlich krankheitserregender Staphylococcus-Arten.

In der DE-A-101 40 772) wird ein Verfahren zur Herstellung von cellulosischen Formkörpern mit inkorporierten Algen beschrieben. Die Formkörper sind in der Lage, aus schwermetallhaltigen Medien Metalle zu adsorbieren. Die schwermetallbeladenen Formkörper können als antibakterielles und/oder fungizides Material verwendet werden. Der Gehalt dieser cellulosischen Formkörper an adsorbierten Schwermetallen ist mindestens etwa 70 mg/kg, vorzugsweise mindestens etwa 200 mg/kg, bevorzugt mindestens etwa 500 mg/kg, insbesondere mindestens 1 000 mg/kg, bezogen auf das Gesamtmasse des cellulosischen Formkörpers. Durch Tauchen von nach Beispiel 1 dieser Veröffentlichung hergestellten Fasern mit einem Braunalgengehalt, bezogen auf die Masse der Fasern, von 11,39 Masse-% in eine 0,05 M AgNO₃-Lösung soll ein Silbergehalt von 1 855 mg/kg Fasern erreicht worden sein. Da Algen Naturprodukte sind, schwanken die relativ begrenzten Bindungskapazitäten für Schwermetalle. Bei der Bindung von Schwermetallen an Algen spielen unterschiedliche Bindungsmechanismen wie Ionentausch, Komplexierung und andere nicht bekannte Reaktionen eine Rolle. Die Bindung der Schwermetalle an den Algen ist deshalb unspezifisch.

Als Trägermaterial werden weiterhin Oxide beschrieben, die mit Silberchlorid imprägniert wurden (WO2004078900) Anti-mikrobielle Lyocellfasern sind aus WO 2006013378 bekannt.

In der EP-A-1 443 099 wird ein Wischtuch beschrieben, bei dem schmelzgesponnene Fasern im Gemisch mit "keramischen" Synthesefasern zu einem Gewebe verarbeitet sind. Die "keramischen" Synthesefasern enthalten Zeolith, an das antibakterielle Zusätze wie Kupfer oder Silber gebunden sind. Infolge der Synthesefasern ist die Wärmebeständigkeit gering.

Tücher enthaltendende Lyocellfasern sind aus EP1614790 bekannt.

### Aufgabe der Erfindung

Der Erfindung liegt die Aufgabe zugrunde, ein microbiocid wirkendes Reinigungsstuch zu schaffen, das sich dauerhaft selbst desinfiziert und seine biociden Eigenschaften über einen weiten Temperaturbereich auch nach mehrmaligem Waschen beibehält.

Diese Aufgabe ist erfindungsgemäß durch ein Reinigungsstuch mit den im Anspruch 1 angegebenen Merkmalen gelöst.

Das Reinigungstuch nach der Erfindung ist mit geringen Kosten herstellbar und kann über einen weiten Temperaturbereich von 0 bis 100°C, kurzzeitig bis 130°C verwendet werden. Es verliert seine selbstdesinfizierenden und desinfizierenden Eigenschaften auch nach wiederholtem Waschen nicht. Das Tuch kann für die üblichen Reinigungsarbeiten und auch zur Isolation von heißen Gegenständen gegenüber empfindlichen Oberflächen sowie zur Trennung von mikrobiologisch besiedel-tenFlächen von unbesiedelten Gegenständen verwendet werden. Wegen seiner selbstdesinfizierenden Eigenschaften kommt es bei Benutzung dieses Tuches nicht zur Verschleppung und Verschmierung von mikrobiologischen Keimen.

Die biocid, genauer gesagt microbiocid wirkenden Metallionen können insbesondere aus der Gruppe der Silber-, Zink-, Kupfer- und Zirkonionen ausgewählt sein. Besonders bevorzugt sind Silberionen.

Besonders universell verwendbar ist das Haushaltstuch in Form eines wärmebeständigen Vliesstoffes, insbesondere eines Nadelvlieses.

Das Nadelvlies kann durch Verfestigungsfasern in Form von Polypropylenfasern und/oder Polyesterfasern, Letztere vorzugsweise mit einem Überzug aus Polypropylen, verfestigt sein. Das Nadelvlies bleibt dann formstabil. Seine Gebrauchseigenschaften bleiben auch nach mehrmaligem Waschen und Erhitzen bis 130°C erhalten.

Die Eigenschaften des Tuches können durch eine Beimischung von weiteren funktionellen cellulosischen Fasern an bestimmte Anwendungsfälle angepasst werden. Diese funktionellen cellulosischen Fasern können insbesondere Lyocellfasern mit inkorporierten Scheuerpartikeln oder antistatisch ausge-rüstete Lyocellfasern sein.

Die Verfestigungsfasern können einen Anteil von 5 bis 15 Masse-% ausmachen.

Als sauere Gruppen haben sich Carboxylgruppen eines Kationen-Ionenaustauscher bewährt. Geeignet sind Polymere auf der Basis von Acrylsäurederivaten, insbesondere Polyacrylmethacrylate.

Die Beladung mit Metallionen, insbesondere mit Silber- oder Zinkionen, kann zwischen 50 mg/kg und 100 000 mg/kg, bevorzugt zwischen 1 000 mg/kg und 80 000 mg/kg der modifizierten Lyocellfasern liegen.

Ein Reinigungstuch nach der Erfindung besteht aus einem Grundmaterial aus cellulosischen Fasern, Viskosefasern, Cellulose-Regeneratfasern (wie Modal und MicroModal) oder Naturfasern, z.B. mit einem Titer von kleiner oder gleich 1,7 dtex. Diesem Grundmaterial sind 1 bis 10 Masse-% Lyocellfasern beigemischt, die ein mit saueren Gruppen modifiziertes Polymer enthalten und je nach späterer Verwendung mit 50 mg/kg bis 100 000 mg/kg Silber, Zink oder einem anderen Schwermetall, vorzugsweise von 1 000 mg/kg bis 80 000 mg/kg Lyocellfasern, beladen sind. Als sauere Gruppen eignen sich, wie gesagt, insbesondere Carboxylgruppen eines Kationen-Ionenaustauschers auf der Basis von Acrylsäurederivaten, insbesondere Polyacrylmethacrylaten. Im Reinigstuch werden Konzentrationen von 2 bis 10 000 mg/Kg Silber, Zink oder einem anderen Schwermetall, vorzugsweise 500 bis 5 000 mg/kg , verwendet.

Die bakterizide Wirkung des Reinigungstuches nach der Erfindung bleibt auch nach zehn Wäschen und nach wiederholter Einwirkung von Temperaturen von kurzzeitig bis zu 130°C erhalten. Die biocide Wirkung reicht für eine Selbstdesinfektion bis herab zu Temperaturen um 0°C aus.

Besonders geeignet ist das vorliegende Reinigungstuch in Bereichen, in denen die Benutzung von bakteriell besiedelten Reinigungstüchern zur Verseuchung von Hartoberflächen führen kann, beispielsweise bei der Reinigung von festen Oberflächen in Krankenhäusern, Pflegeeinrichtungen, Lebensmittelverkaufstheken, Kühlhäusern und Lebensmittelverarbeitungsbetrieben.

### Beispiele

Alle Angaben in % sind Masse-%.

### Beispiel 1

85 % Micromodal (Cellulose-Regeneratfaser, 1,0 dtex, Schnittlänge 39,5 mm, 35,8cN/tex), 10 % Polypropylenfaser (6,7 dtex, Schnittlänge 60 mm) und 5 % Cellulosefaser mit Anteilen von ionentauschergebundenen Metallionen als Silberquelle (6,7 dtex, Schnittlänge 37,8 mm, 22,5cN/tex) werden zu einem Mischvlies mit 50 Einstichen/cm2 mechanisch verfestigt. Nach einer thermischen Verfestigung bei 180 °C beträgt das Flächengewicht 240 g/m2.

### Beispiel 2

80 % Micromodal (Cellulose-Regeneratfaser, 1,0 dtex, Schnittlänge 39,5 mm, 35,8cN/tex), 10 % Polypropylenfaser (6,7 dtex, Schnittlänge 60 mm)und 10 % Cellulosefasern mit Anteilen von ionentauschergebundenen Metallionen als Silberquelle (6,7 dtex, Schnittlänge 37,8 mm, 22,5cN/tex) werden zu einem Mischvlies mit 50 Einstichen/cm2 mechanisch verfestigt. Nach einer thermischen Verfestigung bei 180 °C beträgt das Flächengewicht 240 g/m2.

Cellulosefasern mit Anteilen von ionentauschergebundenen Silberionen können insbesondere nach dem Aminoxid-Verfahren (z.B. US-A-4 246 221 und EP-A-1 358 371) hergestellt werden und sind unter der Bezeichnung "Smart bioclean" im Handel erhältlich.

## Patentansprüche

1. Selbstdesinfizierendes Reinigungstuch aus cellulosischen Fasern als Grundmaterial und modifizierten Lyocellfasern, die mindestens ein mit saueren Gruppen modifiziertes Polymer enthalten, und mit bakterizid wirkenden Metallionen beladen sind.

2. Reinigungstuch nach Anspruch 1, **dadurch gekennzeichnet, dass** die Metallionen aus der Gruppe der Silber-, Zink-, Kupfer-, Zirkon- und Quecksilberionen ausgewählt sind.

3. Reinigungstuch nach Anspruch 1 oder 2, in Form eines wärmebeständigen Vliesstoffes.

4. Reinigungstuch nach Anspruch 3, **dadurch gekennzeichnet, dass** der Vliesstoff ein Nadelvlies ist.

5. Reinigungstuch nach Anspruch 4, **dadurch gekennzeichnet, dass** das Nadelvlies durch Verfestigungsfasern in Form von Polypropylenfasern und/oder Polyesterfasern mit einem Überzug aus Polypropylen verfestigt ist.

6. Reinigungstuch nach einem der Ansprüche 1 bis 5, mit einer Beimischung von weiteren funktionellen cellulosischen Fasern.

7. Reinigungstuch nach Anspruch 6, **dadurch gekennzeichnet, dass** die weiteren funktionellen cellulosischen Fasern Lyocellfasern mit inkorporierten Scheuerpartikeln oder antistatisch ausgerüstete Lyocellfasern sind.

8. Reinigungstuch nach einem der Ansprüche 5 bis 7, **gekennzeichnet durch** einen Anteil der Verfestigungsfasern von 5 bis 15 Masse-%.

9. Reinigungstuch nach einem der Ansprüche 1 bis 8, **dadurch gekennzeichnet, dass** die saueren Gruppen Carboxylgruppen von Kationen-Ionenaustauschern umfassen.

10. Reinigungstuch nach Anspruch 9, **dadurch gekennzeichnet, dass** die Kationen-Ionenaustauscher Polymere auf der Basis von Acrylsäurederivaten, insbesondere von Polyacrylmethacrylaten, sind.

11. Reinigungstuch nach einem der Ansprüche 1 bis 10, **gekennzeichnet durch** eine Beladung der modifizierten Lyocellfasern mit Metallionen (Silber oder Zink) von 50 mg/kg bis 100 000 mg/kg, vorzugsweise von 1 000 mg/kg bis 80 000 mg/kg Lyocellfasern.

12. Reinigungstuch nach einem der Ansprüche 1 bis 11, **gekennzeichnet durch** einen Gehalt an Silber, Zink oder einem anderen Schwermetall im Konstrukt des Reinigungstuchs von 2 bis 10 000 mg/Kg, vorzugsweise 500 bis 5 000 mg/kg.

## Claims

1. A self-disinfecting cleaning cloth made of cellulosic fibers as the base material and modified lyocell fibers which contain at least one polymer modified with acidic groups and which are loaded with metal ions acting in a bactericidal manner.

2. A cleaning cloth according to claim 1, **characterized in that** the metal ions are chosen from the group of silver, zinc, copper, zirconium and mercury ions.

3. A cleaning cloth according to claim 1 or 2, in form of a heat-resistant nonwoven fabric.

4. A cleaning cloth according to claim 3, **characterised, in that** the nonwoven fabric is a needle-punched nonwoven.

5. A cleaning cloth according to claim 4, **characterized in that** the needle-punched nonwoven is strengthened by strengthening fibers in form of polypropylene fibers and/or polyester fibers with a cover made of polypropylene.

6. A cleaning cloth according to one of the claims 1 to 5, comprising an admixture of further functional cellulosic fibers.

7. A cleaning cloth according to claim 6, **characterized in that** the further functional cellulosic fibers ar lyocell fibers with incorporated abrasive particles or antistatic-equipped lyocell fibers.

8. A cleaning cloth according to one of the claims (5 to 7, **characterized by** a content of strengthening fibers of 5 to 15 percent by mass.

9. A cleaning cloth according to one of the claims 1 to 8, **characterized** the acidic groups comprise carboxyl groups of cationic ion exchangers.

10. A cleaning cloth according to claim 9, **characterized in that** the cationic ion exchangers are polymers on the basis of acrylic acid derivatives, especially polyacryl methacrylates.

11. A cleaning cloth according to one of the claims 1 to 10, **characterized by** a loading of the modified lyocell fibers with metal ions (silver or zinc) of 50 mg/kg to 100,000 mg/kg, preferably 1,000 mg/kg to 80,000 mg/kg of lyocell fibers.

12. A cleaning cloth according to on of the claims 1 to 11, **characterized by** a content of silver, zinc or anther heavy metal in the construct of the cleaning cloth from 2 to 10,000 mg/kg, preferably 500 to 5,000 mg/kg.

## Revendications

1. Chiffon de nettoyage auto-désinfectant à base de fibres de cellulose constituant le matériau de base et de fibres de lyocell modifiées, contenant au moins un polymère modifié avec des groupements acides et chargées d'ions métalliques ayant une action bactéricide.

2. Chiffon de nettoyage selon la revendication 1, **caractérisé en ce que** les ions métalliques sont choisis dans le groupe des ions d'argent, de zinc, de cuivre, de zirconium et de mercure.

3. Chiffon de nettoyage selon la revendication 1 ou 2, prenant la forme d'un non-tissé résistant à la chaleur.

4. Chiffon de nettoyage selon la revendication 3, **caractérisé en ce que** le non-tissé est un feutre aiguilleté.

5. Chiffon de nettoyage selon la revendication 4, **caractérisé en ce que** le feutre aiguilleté est renforcé par des fibres de renfort formées de fibres de polypropylène et/ou de fibres de polyester enduites de polypropylène.

6. Chiffon de nettoyage selon l'une des revendications 1 à 5, additionné d'autres fibres de cellulose fonctionnelles.

7. Chiffon de nettoyage selon la revendication 6, **caractérisé en ce que** les autres fibres de cellulose fonctionnelles sent des fibres de lyocell avec des particules abrasives incorporées ou des fibres de lyocell avec un traitement antistatique.

8. Chiffon de nettoyage selon l'une des revendications 5 à 7, **caractérisé en ce qu'**il contient une proportion de fibres de renfort de 5 à 15 % de la masse.

9. Chiffon de nettoyage selon l'une des revendications 1 à 8, **caractérisé en ce que** les groupements acides comprennent des groupements carboxyle d'échangeurs de cations.

10. Chiffon de nettoyage selon la revendication 9, **caractérisé en ce que** les échangeurs de cations sont des polymères à base de dérives décide acrylique, en particulier de polyméthacrylates acryliques.

11. Chiffon de nettoyage selon l'une des revendications 1 à 10, **caractérisé en ce que** les fibres de lyocell modifiées sont chargées avec des ions métalliques (argent ou zinc) à raison de 50 mg/kg à 100 000 mg/kg, de préférence de 1000 mg/kg à 80 000 mg/kg de fibres de lyocell.

12. Chiffon de nettoyage selon l'une des revendications 1 à 11, **caractérisé en ce que** la structure du chiffon de nettoyage contient une teneur en argent, en zinc ou en un autre métal lourd de 3 à 10 000 mg/kg, de préférence de 500 à 5000 mg/kg.
